# EUROPEAN PATENT SPECIFICATION

(11) **EP 2 898 084 B1**
(45) Date of publication and mention of the grant of the patent: **06.01.2021**
(21) Application number: 13770815.2
(22) Date of filing: 20.09.2013
(51) Int. Cl.: C12P 19/02, C12P 19/20, C12P 19/16, C12P 19/14, C13K 1/08, A23L 29/30

(54) **PROCESS FOR INCREASING YIELD OF DEXTROSE PRODUCTION PROCESS, BY MEMBRANE TECHNOLOGY**
VERFAHREN ZUR ERHÖHUNG DES ERTRAGS VON DEXTROSEPRODUKTIONSPROZESSEN DURCH MEMBRANTECHNOLOGIE
PROCÉDÉ PERMETTANT D'AUGMENTER LE RENDEMENT DU PROCESSUS DE PRODUCTION DE DEXTROSE, PAR TECHNOLOGIE DE MEMBRANE

(30) Priority: 24.09.2012 EP 12006676
(43) Date of publication of application: 29.07.2015
(73) Proprietor: Cargill, Incorporated, Wayzata, MN 55391 (US)
(72) Inventor: KETSMAN, Joost, B-9700 Oudernaarde (BE); NATALONI, Luigi, I-40139 Bologna (IT); SANCHEZ, Samuel, Cap 44121 Ferrara (FE) Italy (IT); BANDINI, Serena, I-40131 Bologna (IT)
(74) Representative: Jantschy, Jasmin
(86) International application number: PCT/US2013/060865
(87) International publication number: WO 2014/047418

(56) References cited:
- WO-A1-99/27124
- WO-A2-03/068976
- DE-A1-102009 028 549
- GB-A- 2 129 806
- JP-A- H0 269 190
- US-A- 3 720 583
- US-A1- 2003 092 136
- US-B1- 6 177 265

## Description

### Field of the Invention

The invention relates to a process for increasing the yield of dextrose production from dextrose containing mother liquor of a crystallisation process by membrane filtration and enzyme treatment of the retentate.

### Background of the Invention

Starch hydrolysates rich in dextrose are primarily used as a raw material in the manufacture of crystalline dextrose. Starch hydrolysates rich in dextrose can be obtained through acid conversion of starch, combined acid-enzymatic conversion or multiple enzyme conversion of starch.

Starch hydrolysates rich in dextrose are usually obtained from starch during enzymatic starch hydrolysis process. A typical process comprises:
- Liquefaction: conversion of the starch molecules to dextrose oligomers by addition of an alpha-amylase enzyme to a starch milk, by which the starch milk is converted into a 'liquefied starch milk';
- Saccharification: conversion of dextrose oligomers contained in the liquefied starch milk to dextrose by addition of an amyloglucosidase or glucoamylase enzyme, which converts the liquefied starch milk into a 'saccharified starch milk'.

After saccharification, the starch hydrolysate contains high amounts of dextrose. Crystalline dextrose, having a purity above 90w/w%, can be recovered by crystallisation of this hydrolysate. The by-product of this recovery process is the mother liquor. This mother-liquor is typically sold as a low quality product at reduced price to the feed industry. However, the mother liquor still contains high amounts of dextrose and dextrose oligomers. This high by-product formation negatively affects the efficiency of dextrose production and recovery processes. The dextrose recovery of conventional processes is unsatisfactory.

US 5,869,297 discloses a membrane process to produce substantially pure dextrose, with purity well over the 99%, nanofiltering a glucose syrup containing about 95% dextrose and 5% di- and trisaccharides. Crosslinked aromatic polyamide membranes were used. It discloses glucose syrups having a solids content of about 80 to 97% by weight dextrose and at least 2% di- or trisaccharides.

US 6,126,754 describes a process for the manufacture of a starch hydrolysate with high dextrose content.

WO2007/138167 describes the design of nanofiltration processes for the separation and recovery of sugars at low molecular weight from polysaccharides.

There is a need for an economically-competitive process to increase the yield of current dextrose production processes with a further twofold purpose: decreasing the amount of by-products and enhancing their quality to achieve a commercially attractive purity in dextrose.

### Summary of the Invention

The present invention relates to a process comprising membrane filtration of a dextrose containing mother liquor of a dextrose crystallisation process and treatment of the retentate resulting from the filtration, with one or more glucoamylase and/or pulullanase enzyme.

The present invention further relates to the use of membrane filtration and enzyme treatment of the retentate to increase the dextrose recovery from a dextrose containing mother liquor of a dextrose crystallisation process.

### Description of the drawings

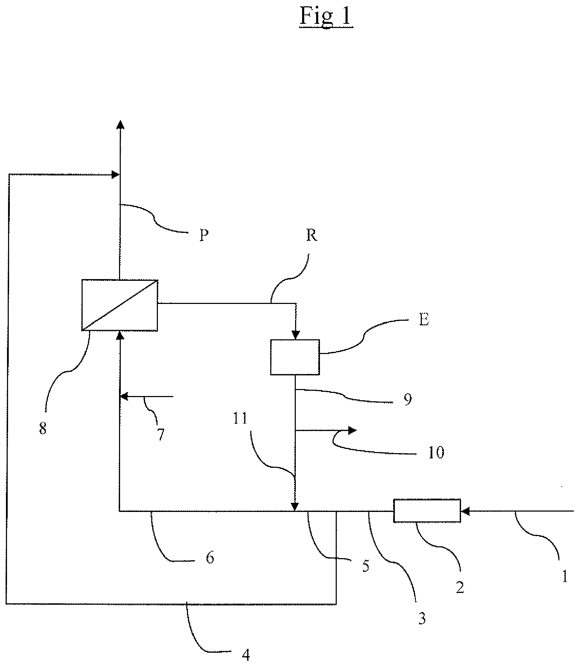

The present invention is further illustrated by the accompanying drawing (Fig 1) which is a flow chart of a pilot system of the invention.

### Detailed Description

The present invention relates to a process comprising membrane filtration of a dextrose containing mother liquor of a dextrose crystallisation process and treatment of the retentate resulting from the filtration, with one or more glucoamylase and/or pulullanase enzyme.

The process of the present invention can be implemented in a dextrose production process. Such process typically comprises starch hydrolysis through liquefaction and saccharification steps:
- Liquefaction: conversion of the starch molecules to dextrose oligomers by addition of an alpha-amylase enzyme to the starch milk. During liquefaction, the starch milk is converted into a 'liquefied starch milk';
- Saccharification: conversion of dextrose oligomers present in the liquefied starch milk to dextrose by addition of amyloglucosidase or glucoamylase enzyme ('original saccharification enzyme'). During saccharification, the liquefied starch milk is converted into a 'saccharified starch milk'.

### Dextrose containing solution

The dextrose containing mother liquor of a dextrose crystallisation process is the feed to the membrane unit.

The dextrose containing solution for the purpose of the present invention is a dextrose containing mother liquor of a dextrose crystallisation process.

The dextrose recovery step comprises a crystallisation. Crystallisation can be done by any suitable crystallisation method known in the art, such as for example batch or continuous crystallisation. After crystallisation, dextrose crystals are recovered by any suitable method known in the art, such as centrifugation, to yield crystalline dextrose and a by-product, called mother liquor. This mother liquor is the dextrose containing solution for the purpose of the present invention. Typically, the mother liquor has a dry substance of from 30 to 60 w/w %. Typically, the mother liquor still comprises from 70 to 85 w/w % ds of dextrose, preferably from 75 to 83 w/w % ds of dextrose.

The by-product formed after crystallisation usually still comprises high amounts of dextrose and starch hydrolysis products other than dextrose such as disaccharides, trisaccharides and oligosaccharides. Such by-product can also comprise the saccharification enzymes, originally present in the saccharified starch milk. In one aspect the enzyme originally present in the saccharified starch milk has been deactivated throughout the process. In another aspect the saccharification enzyme has not been deactivated throughout the process and remains active in the by-product.

Further, the dextrose containing mother liquor of a dextrose crystallisation process can be refined before the nanofiltration ('refined dextrose containing solution'). Purification can comprise removal of salts and/or protein by resin treatment such as to bring the conductivity of the dextrose containing solution below 50µS/cm. Resin can be for example cation-anion system.

### Membrane filtration

Filtration membranes can be classified by their porous vs. nonporous structure. Alternatively, membrane processes can be identified on the basis of the main driving force, such as pressure difference, concentration difference, etc. Pressure-driven membrane separation processes for liquid mixtures are reverse osmosis, nanofiltration, ultrafiltration and microfiltration. Membrane performances greatly depend on operative conditions of pressure, temperature, composition and pH, whereas the process efficiency is also affected by the fluid-dynamic conditions existing in the modules, by the configuration of the membrane filtration unit (single-pass, feed and bleed etc.) and the module arrangement can be relevant as well. Filtration membranes can be characterized by their cut-off value. Membrane cut-off value can either be expressed in terms of particle size (particle size cut-off value) or in terms of molecular weight (molecular weight cut-off value). The maximum value, either in particle size or in molecular weight that a particle can have in order to pass the filtration membrane determines the cut-off value of the filtration membrane. Filtration processes result in the production of a permeate and a retentate. The permeate comprises material having either a particle size or a molecular weight equal to or smaller than the cut-off value of the membrane. The retentate comprises material having either a particle size or a molecular weight greater than the cut-off value of the membrane.

Preferably, the membrane filtration of the present invention comprises nanofiltration; more preferably, the membrane filtration of the present invention is a nanofiltration.

Nanofiltration typically shows separation characteristics which are intermediate between reverse osmosis and ultrafiltation, with some overlap at lower and higher cut off values.

For the process of the present invention, the nanofiltration membrane has a cut-off value which allows passage of dextrose molecules into the permeate while retaining glucose polymer molecules such as disaccharides and trisaccharides into the retentate. Preferably, the nanofiltration membrane has molecular weight cut-off values in the range of from 100 to 400 Dalton, preferably from 200 to 300 Dalton, more preferably from 150 to 200 Dalton. Such membrane are for example commercialised by Koch Membrane Systems or General Electric-Power & Water, also known as Desal membranes. Thus a process comprising nanofiltration of a dextrose containing mother liquor of a dextrose crystallisation process and treatment of the retentate resulting from the filtration, with one or more glucoamylase and/or pulullanase enzyme, wherein the nanofiltration allows passage of dextrose molecules into the permeate while retaining glucose polymer molecules such as disaccharides and trisacharides into the retentate is described.

Suitable modules are in tubular and/or in spiral wound configuration, in order to prevent concentration polarization phenomena in the feed side. Spiral wound modules are preferable showing feed spacers in the range 30-70 mil, preferably in the range 30-50 mil, more preferably at 50. Suitable modules are for example Module KOCH-4720SR2-N1, Module KOCH-MPS34A2Z, Module GE-DK4040C1027 , Module GE-DL4040C1025, with GE-DL8040C being even more suitable.

Preferably, the nanofiltration is carried out under temperature condition of from 30°C and 60°C, preferably about 50°C, and pressure conditions are preferably in the range of from 15 and 35 bar, more preferably 25 to 30, even more preferably about 30 bar. pH is preferably in the range of from 3.5 to 5, more preferably in the range of from 4.2 to 4.5.

The permeate has a dextrose purity which is higher than the dextrose purity of the dextrose containing mother liquor of a dextrose crystallisation process. It preferably comprises from 80 to 99 w/w % ds, more preferably from 90 to 99 w/w % ds, even more preferably from 95 to 99 w/w % ds of dextrose (dextrose purity). In one aspect, the dry substance of the permeate is from 10 to 40w/w%, preferably from 20 to 35w/w% The permeate can be further treated to recover the dextrose, for example by crystallisation. Alternatively, the permeate can be concentrated and either used as a dextrose syrup or as a raw material for crystallisation to produce crystalline dextrose powder, or for fructose production. Alternatively, the permeate can be concentrated and sent to a hydrogenation process for production of polyols.

The dextrose purity of the retentate can be from 1 to 99 w/w % ds, more preferably from 50 to 80 w/w % ds, even more preferably from 60 to 75 w/w % ds of dextrose. The dry substance of the retentate can be from 10 to 50 w/w%, it can be from 30 to 45 w/w %, it can be from 35 to 40 w/w %.

### Enzyme treatment

The present invention relates to a process comprising membrane filtration of a dextrose containing mother liquor of a dextrose crystallisation process, and an enzyme treatment of the retentate with one or more glucoamylase and/or pulullanase enzyme.

The dextrose content of the retentate is increased through the action of the glucoamylase and/or pulullanase enzyme, converting the glucose oligomers present in the retentate into dextrose.

The enzyme treatment can be achieved in different ways. Fresh enzyme can be added to the dextrose containing solution once or several times during a continuous process. Said enzyme is retained by the filtration membrane. In this way the enzyme converts the recycled retentate.

In a preferred embodiment, the enzyme is immobilized and/or adsorbed on the filtration membrane itself.

In another preferred embodiment, the enzyme is immobilized and/or adsorbed on an fixed phase system like a resin through which the retentate passes. Suitable resins are for example anionic, macroporous or phenolic resins. Suitable phenolic resin is for example Duolite A 568. The fixed phase system is suitably placed subsequent to the filtration membrane.

The temperature is chosen to be in the optimal range for the enzyme. In the case of a glucoamylase, a suitable temperature range is of 50 to 65°C. The pH of the retentate depends on the enzyme used. It is preferably in the range of from 3.5 to 5, more preferably in the range of from 4.2 to 4.5.

During the enzymatic conversion the percentage of dextrose in the retentate may be adjusted by varying the contact time between the retentate and the enzyme, dry substance of the retentate and the temperature of the reaction.

Through the action of the glucoamylase and/or pulullanase enzyme, the dextrose content of the retentate is increased. Preferably, it reaches the dextrose content level of the dextrose containing solution.

The enzymatically treated retentate is recycled and is used as part of the mother liquor of a dextrose crystallisation process. It is thus passed through a filtration membrane, to yield a retentate and a permeate. This retentate can be treated with enzyme and recycled in turn. This recycling can be repeated as many times as needed, to finally recover substantially all dextrose from the dextrose containing solution. At least 30% of the retentate can be recycled, preferably at least 50%, more preferably at least 70%, even more preferably at least 90% and most preferably 100% of the retentate is recycled. Thereby, the by-product formation of the dextrose production process is significantly decreased in amount and can even be eliminated. Thus in a most preferred aspect, the process has one inflow stream, the dextrose containing mother liquor of a dextrose crystallisation process and one outflow stream, the permeate, while the retentate is recycled. The present invention allows to achieve yield of at least 80%, preferably at least 90%, more preferably at least 95%. It is understood that the yield for the purpose of the present invention is the ratio between the dry matter of the permeate on the dry matter of the dextrose containing mother liquor of a dextrose crystallisation process.

Thus in the process of the present invention the dry substance of the permeate is at least 80%, preferably at least 90%, more preferably at least 95% of the dry substance of the dextrose containing mother liquor.

Additonally, after the enzymatic treatment, the retentate can be demineralised with resin columns for example in order to remove ionic content and proteins. The demineralised, enzymatically treated retentate is then recycled to the membrane filtration.

The process of the present invention can be batch, continuous or semi-continuous.

The dextrose producing process preferably relates to a process comprising the following steps:
- Liquefaction of a starch milk to produce a liquefied starch milk, and
- Saccharification of the liquefied starch milk through the action of one or more saccharification enzyme to produce a saccharified starch milk containing dextrose, and
- Crystallisation of dextrose and recovery of crystalline dextrose with mother liquor as by-product, and
- Nanofiltration of the mother liquor to obtain a high yield of permeate rich in dextrose and a retentate,
wherein the retentate is enzymatically treated with one or more glucoamylase and/or pulullanase and recycled into the nanofiltration unit.
Optionally the process comprises crystallisation of dextrose out of the permeate.

The present invention also allows working with dextrose containing mother liquor of a dextrose crystallisation process having a high dry substance content. In conventional processes, nanofiltration requires that the incoming dextrose containing solution has a dry substance content of up to 10 to 25 w/w%. At higher dry substance contents, a lot of retentate is produced which means a lot of low quality by-product to be sold at lower price. With the present invention, there is no loss of retentate, there is limited to no by-product formation, because of the recycling of the retentate to the filtration unit. Therefore dextrose containing solution having a dry substance of up to 70w/w% can be used. Conveniently, the dextrose containing solution has a dry substance content of from 20 w/w% to 60 w/w%.

Further, the present invention relates to a use of membrane filtration of a dextrose containing mother liquor of a dextrose crystallisation process and subsequent enzyme treatment of the retentate with one or more glucoamylase and/or pulullanase enzyme to increase dextrose recovery from a dextrose containing solution. Membrane filtration, dextrose containing solution and enzyme treatment are as described above. In conventional processes, dextrose recovery is about 50%. With the process of the present invention, dextrose recovery is greater than 95%.

A pilot system (Fig 1) was set up to test large scale production. The dextrose containing mother liquor of a dextrose crystallisation process (1) enters via a feed pipe and can be pass through a resin (2) to remove salt and protein such as to produce a refined dextrose containing solution (3). Part of the refined dextrose containing solution (4) by-passes the nanofiltration and is mixed with the permeate (P). The rest of the refined dextrose containing solution (5) is supplied with part of the enzyme treated retentate (upgraded retentate) (11) to form a second dextrose containing solution (6) which is diluted with water (7) in a feed tank. This diluted dextrose containing solution is going to the nanofiltration unit (8). The permeate (P) is supplied with part of the refined dextrose containing solution (4). The retentate (R) is sent to a resin with fixed enzyme (E). The upgraded retentate (9) is partly bled out (10) and partly recycled towards the nanofiltration unit (11). This pilot system is one way of carrying out the invention, it should not be understood as limiting the invention.

### Examples

### Example 1: nanofiltration of crystallisation molasses

This example describes a continuous nanofiltration process of mother liquor coming from the centrifugation of dextrose crystallization process. The example is schematically represented in Figure 1.

Nanofiltration (8) is carried out at 50°C, at pH values of 4 and feed pressure conditions of 30 bar. Spiral wound thin film composite membranes Desal GE-DL4040C1025 are used, manufactured by General Electric; general specifications are reported in the following table

**GE-DL4040C1025: general specifications from technical sheets**

| | |
|---|---|
| Typical Operating: | Flux 5 - 20 GFD (8 - 34 LMH) |
| Maximum Operating Pressure | 600psi (4,137kPa) if T < 95°F (35°C) |
| | 435psi (3,000kPa) if T > 95°F (35°C) |
| Maximum Temperature Continuous Operation: | 122°F (50°C) |
| Clean-In-Place (CIP): | 104°F (50°C) |
| pH Range Continuous Operation: | 3-9 |
| Clean-In-Place (CIP): | 2-10.5 |
| Chlorine Tolerance | 500 ppm hours, |
| Feed spacer | 50 mil |
| Active area | 66 ft² (6.1 m²) |
| Average permeate Flow | 1600 GPD (6.0 m³/day) |
| Minimum MgSO₄ rejection | 96% |
| Module Diameter | 3.9 in |
| Module length | 40 in |

The nanofiltration unit operates with a minimum overall volume concentration factor of 2.2 with a surface of 125 m2/tons of diluted mother liquor.

0.39 tons/h of mother liquor coming from the centrifugation of dextrose crystallization process (1), at 50 w/w% dry substance (weight/weight %) and 82w/w% dextrose purity, is refined (de-ashing) in a double pass resin system (cation-anion) (2) to remove salts and proteins. The mother liquor after refining has a conductivity below 50µS/cm (refined mother liquor (3)).

0.033 tons/h of refined mother liquor (5) is mixed with 0.56 tons/h of upgraded retentate (11) coming from the enzyme treatment and having a dry substance of 39w/w% and a dextrose purity of 82w/w%. This provides 0.89 tons/h of diluted mother liquor (6). The 0.89 tons/h of diluted mother liquor is further diluted with 0.39 tons/h of water (7) in a feed tank in order to obtain 1.28 tons/h of further diluted mother liquor at a dry substance of 30 w/w%. The latter is fed to the nanofiltration unit (8). The permeate stream (P) (0.7 tons/h) has a dry substance of 23 w/w%, and a dextrose purity of 98 w/w% and is mixed with 0.06 Tons/h of the refined mother liquor (4) to yield a syrup having a dry substance of 24.8 w/w% and a dextrose purity of 97.3w/w%. This syrup is pumped to the crystallization monohydrate process.

The retentate (R) (0.58 tons/h) has a dry substance of 39 w/w%, and a dextrose purity of 71.5 w/w%. The retentate is fed to a column Duolite568 with bound glucoamylase and pullulanase enzyme in order to convert the polysaccharides into dextrose to increase the dextrose purity level at same level than the nanofiltration feed solution, i.e. 82w/w% ('upgraded retentate' (9)). Conversion occurs within one hour retention time at pH 4-5, at 50°C.

0.56 tons/h of upgraded retentate (11) is recycled to the nanofiltration unit as explained above, and 0.022t/h of the upgraded retentate (10) is sent to the concentrator for downstream utilization.

The example described allows achieving an overall yield of at least 95% tons ds of permeate (P) liquor/tons ds of mother liquor coming from crystallization (1).

## Claims

1. A process comprising membrane filtration of a dextrose containing solution and treatment of the retentate resulting from the filtration with one or more glucoamylase and/or pulullanase enzyme, wherein the dextrose containing solution is a mother liquor of a dextrose crystallisation step.

2. The process according to claim 1, wherein the dextrose containing solution has a dry substance of up to 70w/w%, preferably from 20 to 40w/w%, more preferably from 25 to 35 w/w%.

3. The process according to claim 1 or claim 2, wherein the membrane filtration is nanofiltration having a molecular weight cut-off value of from 100 to 400 Dalton.

4. The process according to any of claims 1 to 3, wherein the enzyme is immobilized on a resin.

5. The process according to any of claims 1 to 3, wherein the enzyme is immobilized on and/or retained by the filtration membrane.

6. The process according to claim 1 to 5, wherein the dry substance of the permeate is at least 80%, preferably at least 90%, more preferably at least 95% of the dry substance of the dextrose containing solution.

7. Use of membrane filtration of a dextrose containing mother liquor of a crystallisation step and subsequent enzyme treatment of the retentate resulting from the filtration to increase the dextrose recovery from a dextrose containing solution, wherein the enzyme is one or more glucoamylase and/or pullulanase enzyme.

## Patentansprüche

1. Verfahren, umfassend Membranfiltration einer Dextrose enthaltenden Lösung und Behandlung des aus der Filtration resultierenden Retentats mit einem oder mehreren Glucoamylase- und/oder Pullulanase-Enzymen, wobei die Dextrose enthaltende Lösung eine Mutterlauge eines Dextrosekristallisationsschritts ist.

2. Verfahren nach Anspruch 1, wobei die Dextrose enthaltende Lösung eine Trockensubstanz von bis zu 70 % (Gew.-/Gew.), vorzugsweise 20 bis 40 % (Gew.-/Gew.), noch bevorzugter 25 bis 35 % (Gew.-/Gew.), aufweist.

3. Verfahren nach Anspruch 1 oder Anspruch 2, wobei die Membranfiltration Nanofiltration ist, die einen Molekulargewicht-Cutoff-Wert von 100 bis 400 Dalton aufweist.

4. Verfahren nach einem der Ansprüche 1 bis 3, wobei das Enzym auf einem Harz immobilisiert ist.

5. Verfahren nach einem der Ansprüche 1 bis 3, wobei das Enzym auf der Filtrationsmembran immobilisiert ist und/oder dadurch zurückgehalten wird.

6. Verfahren nach einem der Ansprüche 1 bis 5, wobei die Trockensubstanz des Permeats mindestens 80%, vorzugsweise mindestens 90%, noch bevorzugter mindestens 95 %, der Trockensubstanz der Dextrose enthaltenden Lösung beträgt.

7. Verwendung von Membranfiltration einer Dextrose enthaltenden Mutterlauge eines Kristallisationsschritts und anschließender Enzymbehandlung des aus der Filtration resultierenden Retentats, um die Dextroserückgewinnung aus einer Dextrose enthaltenden Lösung zu erhöhen, wobei das Enzym ein oder mehrere Glucoamylase- und/oder Pullulanase-Enzyme ist.

## Revendications

1. Procédé comprenant la filtration sur membrane d'une solution contenant du dextrose et le traitement du rétentat résultant de la filtration avec une ou plusieurs enzymes glucoamylase et/ou pulullanase, dans lequel la solution contenant du dextrose est une liqueur mère d'une étape de cristallisation de dextrose.

2. Procédé selon la revendication 1, dans lequel la solution contenant du dextrose contient une substance sèche jusqu'à 70 % en poids/poids, de préférence de 20 à 40 % en poids/poids, plus préférentiellement de 25 à 35 % en poids/poids.

3. Procédé selon la revendication 1 ou la revendication 2, dans lequel la filtration sur membrane est une nanofiltration présentant une valeur seuil de coupure de poids moléculaire de 100 à 400 Dalton.

4. Procédé selon l'une quelconque des revendications 1 à 3, dans lequel l'enzyme est immobilisée sur une résine.

5. Procédé selon l'une quelconque des revendications 1 à 3, dans lequel l'enzyme est immobilisée sur et/ou retenue par la membrane de filtration.

6. Procédé selon les revendications 1 à 5, dans lequel la substance sèche du perméat représente au moins 80 %, de préférence au moins 90 %, plus préférentiellement au moins 95 % de la substance sèche de la solution contenant du dextrose.

7. Utilisation d'une filtration sur membrane d'une liqueur mère contenant du dextrose d'une étape de cristallisation et ensuite d'un traitement enzymatique du rétentat résultant de la filtration pour augmenter la récupération de dextrose à partir d'une solution contenant du dextrose, dans laquelle l'enzyme est une ou plusieurs enzymes glucoamylase et/ou pulullanase.
